(19) Europäisches Patentamt
European Patent Office
Office européen des brevets

(11) **EP 3 238 764 B1**

(12) **EUROPEAN PATENT SPECIFICATION**

(45) Date of publication and mention of the grant of the patent:
**10.11.2021   Bulletin 2021/45**

(51) Int Cl.:
*A61M 1/36* *(2006.01)*     *A61M 60/50* *(2021.01)*
*A61B 5/02* *(2006.01)*     *A61B 5/00* *(2006.01)*
*A61B 5/021* *(2006.01)*

(21) Application number: **17168591.0**

(22) Date of filing: **28.04.2017**

(54) **A METHOD OF DETECTING INCIPIENT BLOOD VESSEL COLLAPSE AND A DEVICE CONFIGURED TO CARRY OUT THE METHOD**

VERFAHREN ZUR DETEKTION EINES BEGINNENDEN BLUTGEFÄSSKOLLAPS UND VORRICHTUNG ZUR AUSFÜHRUNG DES VERFAHRENS

PROCÉDÉ DE DÉTECTION D'AFFAISSEMENT DE VAISSEAUX SANGUINS NAISSANTS ET DISPOSITIF DESTINÉ À METTRE EN  UVRE LE PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **28.04.2016   EP 16461518**

(43) Date of publication of application:
**01.11.2017   Bulletin 2017/44**

(73) Proprietors:
• **Kiluk, Sebastian**
  **32-406 Zakliczyn (PL)**
• **Szuldrzynski, Konstanty**
  **30-069 Krakow (PL)**

(72) Inventors:
• **Kiluk, Sebastian**
  **32-406 Zakliczyn (PL)**
• **Szuldrzynski, Konstanty**
  **30-069 Krakow (PL)**

(74) Representative: **AOMB Polska Sp. z.o.o.**
  **Ul. Emilii Plater 53**
  **21st Floor**
  **00-113 Warsaw (PL)**

(56) References cited:
  **WO-A1-92/02264**     **WO-A2-02/35979**
  **US-A1- 2002 099 286**

## Description

### Technical field

This invention relates to a method for monitoring and optimizing automatically the blood flow of a pump in a manner that prevents blood vessel collapse.

Conclusions can be drawn from the control parameters as to the patient's condition and any problems in the blood circulation generated by the pump. This ensures that the blood vessel from which the blood is drawn into the extracorporeal circuit does not collapse and an adequate blood flow takes place at all times.

The invention also relates to a medical apparatus which comprises a pump control system according to this method.

### Background

Blood pumps and associated control systems have long been known in the prior art. The task of blood pumps in combination with associated control systems is to ensure blood circulation intra- or extra-corporeally and thus to support or replace the pumping function of the patient's heart. Such blood pumps are used for a limited time to replace the heart function during operations on the heart or to support the heart function as a possible means for recovery of the weak heart in an extracorporeal circuit. These pumps may also support or replace the function of lungs providing blood flow through the extracorporeal circuit equipped in an oxygenator, in which extracorporeal gas exchange takes place.

Apart from the blood pump, use may be made, depending on the requirements, of all products commonly used for the extracorporeal circuit, such as for example cannulas, catheters, tubes and connectors, reservoirs, oxygenators, heat exchangers, blood concentrators and dialyzers, bubble traps and filters.

Moreover, applications of heart and/or lung support for an unlimited time and also artificial hearts and lungs are known or possible.

In all these applications of blood pumps, it is of utmost importance to generate reliably a sufficient blood flow by means of the pump. Especially in the case of the longer-term use of the blood pump, it is not possible manually to monitor and, if need be, correct the blood flow and associated parameters such as venous or arterial blood pressure or gas flow and gas mixture with the simultaneous use of oxygenators. At the same time, collapse of venous vessel from which the blood is drawn into the extracorporeal circuit leads to the cessation of flow and exposes blood on the increased risk of mechanical injury by the pump rotor rotating in the stationary blood.

A reliable automatic control of the blood pump is therefore required especially for the use of ECMO (extracorporeal membrane oxygenation) or ECLS (extracorporeal life support), since these applications take place over a fairly lengthy period of days up to weeks and also outside the operating theatre and without staff-intensive monitoring. But even in the case of applications in heart surgery, an automatic pump control offers greater convenience in handling and greater safety in the case of flow disruptions. Moreover, such a control is also conceivable in the case of implantable support systems (ventricular assist devices, VAD's) or in the case of an implantable artificial heart.

In the following, the factors are dealt with that adversely affect a desired blood flow, so that the pump settings and possibly additional parameters have to be changed.

For the venous blood return flow, it is known from practice and from Baloa et al. [1], Vollkron et al. [2] that, in the case of insufficient venous return flow of the blood, the large venous vessels and/or the atrium of the heart (depending on which one is being drained, the left or the right atrium) may collapse and then no blood flow at all or only an insufficient blood flow is possible. This venous return flow depends, amongst other things, on the filling status and the position and construction of the venous catheter. In the case of an excessively small filling status or in the event of contact of the catheter opening with the vessel wall, the vessel collapses and obstructs the venous and therefore the whole blood flow. This phenomenon is known in connection with centrifugal pumps, but can occur independently of the pumping principle. Furthermore, impairments of the venous return flow due to thrombosis or suchlike or kinking of the tube in the venous line are conceivable. If such a state of insufficient venous return flow is reached, not only insufficient blood flow but also damage to the blood can occur due to cavitation and formation of the gas bubbles. This life-threatening and patient-endangering state therefore has to be reliably avoided.

The monitoring of the pressure on the venous line is not sufficient to avoid collapse, because negative pressures can occur both in the case of collapse as well as in the operationally safe state. Moreover, the pressure on the venous side of the blood pump is to a large extent dependent on its position in relation to the patient (hydrostatic pressure) and can therefore be influenced thereby.

Furthermore, a collapse that has already occurred can at best be detected in this way and the tendency towards collapse can only be detected with difficulty.

In the arterial line, it is exclusively obstructions or closures due for example to thromboses or embolisms or kinking of the tube that are conceivable as an impairment of the blood flow. The problem of the collapse of the vessel wall does not exist on account of the arterial pressure. The level of the arterial pressure alone is not suitable as a measure for the detection of flow disruptions, because a high arterial flow resistance can be caused by the stated flow obstructions in the arterial line or by a high vascular resistance.

Approaches are disclosed in the prior art which characterize this problem mathematically. Baloa et al. disclose for example the method of forming the differential variable DRI (diminishing return index) DRI=dQ/dω by gradually increasing the pump speed, measuring the respective flow and differentiating by the pump speed. In the ideal case of unobstructed flow, this variable is a constant. With an incipient collapse, DRI becomes smaller, reaching the value 0 when collapse occurs. It has not yet been however disclosed how a possible collapse is to be detected. The method employed in many VAD's on the market of simply switching off the pump for several seconds in such cases appears to involve a high degree of risk due to the sudden total removal of the pump function and therefore the circulation support. In this case, the weakened patient's heart suddenly has to take over the whole burden of the blood circulation without a process of adaptation, and this can lead to it being overloaded and therefore to additional damage. In addition, the collapse can reoccur at any time with the described consequences when the original speed is restored. If, for the sake of safety, a lower pump speed is instead preset, the smaller flow thereby generated can possibly be maintained in a stable manner. Then, however, there is a reduced flow compared to the basic setting, said flow not being able to produce the full intended circulation support. In addition, the application described by Baloa et al. is used in a VAD, wherein it is not a vessel, but rather the atrium that collapses.

The disclosure of Simons [3] suggests that increasing blood flow periodically can detect the limitations of blood flow and incipient blood vessel collapse by disruption of the ratio of pump speed to the blood flow. Then reducing the pump speed can restore the previous ratio preventing blood vessel collapse. Unfortunately sudden blood vessel collapse may arise from the change of patient's position, which cannot be detected soon enough, with the means of the technology developed by Simons.

Acoustic detection techniques and apparatus described by Sandler and Mansy [4] enable the non-invasive assessment of vascular conditions within a human or animal body. Generally speaking, the acoustic detection techniques and apparatus described by Sandler and Mansy measure vibrations or sounds generated by blood flowing through shunts, arteries and/or veins and process these measured vibrations or sounds to diagnose the internal condition of the shunts, arteries and/or veins. In particular, the acoustic detection techniques and apparatus described may be used to assess vascular patency in, for example, an AV shunt that provides vascular access for dialysis procedures. Thus, the acoustic detection techniques and apparatus described may be used to determine if a critical AV shunt has failed or is near failure, thereby reducing the possibility that a dialysis patient will be subjected to a life threatening condition.

Therefore the problem underlying the present invention is to make available a device or a computer program and a method for automatically regulating and optimizing, within the range of the desired preset values, the blood flow from blood pumps whilst avoiding life-threatening states, wherein the method is intended to be universally usable, i.e. one that functions reliably even in the event of cardiac arrest, and in the case of corrected impairments of the blood flow it should be possible to detect their location in the circulation, venous or arterial, in order to permit rapid measures to be taken in a targeted manner.

This problem is completely solved by the method according to the invention and a device or the computer program for performing the method.

## Summary

In the present solution detection of the first symptoms of vein collapse, realized through the analysis of the pressure/flow oscillations (a pre-collapse oscillation state) preceding total collapse with concomitant sudden pressure drop in the blood-collecting part of the circuit, is based on detection of short, repetitive changes of blood pressure measured on the inlet side of the pump.

The volume of the blood inflow must be equal to the sum of the blood volume drained to the circuit, usually extracorporeal, and outflowing in the section of the vein where the drainage cannula is located. Due to the incompressible nature of blood and the elastic characteristic of veins suspended in the soft tissues of body the deficit of the blood inflow to the section of vein may be temporarily compensated by reduction of the volume of the section of vein where the cannula is positioned by drawing the vein walls nearer. As a result of the inflow deficit the diameter of the vein is locally decreased and the negative pressure generated on the inlet side of the pump is rising till the moment when the opposite walls of the vein come into contact closing lumen of the vein and blocking blood flow. The inflow of blood to the collapsed segment of vein together with the elastic forces of vein walls and surrounding tissues result in the reopening of the vein lumen re-establishing the blood flow. Cyclic recurrence of this phenomenon will cease when the cause of blood inflow deficit is eliminated as for example through decreasing the output of the pump. The changes of the blood flow in the extracorporeal circuit at the given pump output are accompanied by the pressure changes measured in the circuit. Especially, the cyclic flow changes lead do detectible pressure changes in the pump circuit, as for example on the inlet side. The characteristic of the time variability of the signal and/or its frequency characteristic allow for unambiguous differentiation of the signal compound generated by the cyclic changes of flow from the changes resulting from patients activity, circulatory system physiology or operation of the extracorporeal circuit under normal conditions.

Therefore, the incipient collapse may be prevented regardless of the cause: increased pump speed, inadequate vascular filling or patient's position changes. It is also possible to evoke the characteristic pattern of the pressure waveform by periodically increasing the blood flow. In this way an upper flow limit may be determined. After determining the flow limit the pump speed may be decreased to the safe values. The pulsatility that was observed by the inventors is produced by the inadequate filling of the inflow cannula and has nothing to do with the beating heart.

The method according to the invention is capable of detecting incipient vessel collapse by analysis of the pressure waveform and seeking for the characteristic pattern that was observed.

The pre-collapse oscillation state may alternatively be detected by monitoring and analyzing vibrations of cannula (or other means for drainage), the power of the pump, a vibro-acoustic characteristic of the pump-blood-vessel dynamic system, or any other detectable manifestation of the pre-collapse oscillation state on the inlet or on the outlet side of the pump.

This new method can also automatically perform periodic interventions into the pump speed, wherein the flow limit is ascertained by determining the level of flow, at which a pulsatile effect takes place.

The blood flow required depending on the patient's needs is established in a known manner by the doctor in charge or perfusionist and the numerical magnitude "% of the blood flow required in the circulation" required for the method according to the invention is thus also known. For example, the required blood flow can be calculated using the du Bois formula for a patient-related body area, cardiac indices in the range of 2.5-41 min-1 m-2 being normal.

In contrast to Simons a tendency towards collapse is detected not only by increase of the pump speed but every time a characteristic waveform pattern is observed irrespective of the pumps speed/flow ratio. In such an instance a pump speed is reduced to prevent collapse and to achieve a sufficient venous return flow.

The method according to the invention, wherein an incipient collapse is not only detected, but also rapidly eliminated and the flow thereafter optimized, differs markedly from the prior art (Simons, Sandler and Mansy) as it is irrespective of the pump speed/flow ratio. The method employed in many extracorporeal circulation devices on the market of simply switching off the pump for several seconds in such cases appears to involve a high degree of risk due to the sudden total removal of the pump function and therefore the circulation support.

The invention is defined by the appended claims.

According to the invention, a method of detecting incipient blood vessel collapse in a circuit comprising means for drainage, preferably a cannula, positioned in said vessel and connected to a pump, is characterized in that the incipient vessel collapse is detected by detecting a pre-collapse oscillation state characterized by oscillations of blood pressure of substantially constant frequency and limited amplitude.

The pre-collapse oscillation state is further characterized by the oscillation frequency $f_{osc}$ between 0,1Hz and 10Hz and substantially steady mean value of the blood pressure.

Preferably the pre-collapse oscillation state is further characterized in that a pump speed change causes change of the amplitude of the oscillations, whereas the mean value of the blood pressure remains substantially steady.

A pre-collapse oscillation state may be detected by monitoring and analyzing vibrations of the means for drainage, power of the pump and/or a vibro-acoustic characteristic of the pump-blood-vessel dynamic system.

Preferably a pre-collapse oscillation state is detected by monitoring the blood pressure on the inlet side of the pump and detecting the oscillations of the blood pressure.

Preferably a pre-collapse oscillation state is detected by means for detecting any suitable parameter corresponding to any manifestation of the pre-collapse oscillation state, for example a pressure sensor on the inflow line for monitoring the blood pressure on the inlet side of the pump, accelerometer or a camera equipped with the means for image analysis for monitoring vibrations of the means for drainage.

Detection of the oscillations may include one or more of following methods:

- frequency domain analysis such as Fast Fourier transform (FFT) for detection of the frequency close to $f_{osc}$ and preferably also for detection of the amplitude changes according to pump speed change;
- time domain analysis of time series for detection of the steady mean value of the blood pressure, and preferably also for detection of the amplitude changes according to pump speed change;
- statistical analysis of residuals filtered from pressure time series with high-pass filter filtering out mean value with time constant $T_{osc}=1/f_{osc}$ utilising the segmentation of the residuals with statistical reasoning on the significance of residuals variation change;
- wavelets for detecting typical signal shapes occurring during beginning of the pre-collapse oscillations state and corresponding to the main frequency of the pump-blood-vessel dynamic system or group frequencies;
- artificial neural networks trained to detect oscillations in the vessel blood pressure time series.

An object of the invention is also a device or a computer program configured to carry out a method of optimizing the blood flow of a pump in a circuit comprising means for drainage, preferably a cannula, positioned in said vessel and connected to the pump, characterized in that the method comprises the steps of:

a) estimation of expected maximum pump speed $S_{max}$ and minimum vessel blood pressure $P_{min}$ allowed for a given therapy,

b) estimation of the oscillation length $T_{osc}=1/f_{osc}$ and an pressure zone of the pre-collapse oscillations state $P_{zone} = [P_{zone1}, P_{zone2}]$, wherein said pressure zone $P_{zone}$ is a blood pressure range of width $\Delta P = |P_{zone2} - P_{zone1}|$, in which the pre-collapse oscillations state is expected to be observed, and wherein $P_{zone1} > P_{min}$, $P_{zone2} > P_{min}$,

c) estimation of the maximum rate of non-oscillating blood pressure change $V_{max}= \Delta P/T_{osc}$ and the maximum rate of pump speed change rate $R_{max} = |k(P_{zone2})-k(P_{zone1})|/T_{osc}$, wherein k(P) is a speed-pressure pump characteristics,

d) introducing controlled pump speed increase at rate $R<R_{max}$ with simultaneous control of the blood pressure P and the blood pressure change rate V, wherein the pump speed S is increased until:

> the pump speed $S = S_{max}$ is reached, or
> the blood pressure $P =P_{min}$ is reached, or
> an incipient blood vessel collapse is detected by the method according to any of the preceding claims, or
> the blood pressure change rate V exceeds $V_{max}$,

and wherein the pump speed is temporarily decreased if the pressure change rate V exceeds $V_{max}$ until the pressure change rate V does not exceed $V_{max}$, and then is further increased,

e) setting new maximum pump speed $S_{max} = S_{osc}$ if the incipient blood vessel collapse is detected for the pump speed $S = S_{osc}$ before maximum pump speed $S_{max}$ is reached ,or

setting new maximum pump speed $S_{max} = S_{Pmin}$ if the blood pressure $P =P_{min}$ is reached for the pump speed $S = S_{Pmin}$ before maximum pump speed $S_{max}$ is reached.

Preferably the method step e) further comprises the step of increasing maximum pump speed $S_{max}$ with a predetermined value, if $S = S_{max}$ is reached during the method step d).

The steps d) and e) may be performed periodically or on demand of the operator to refresh the maximum pump speed settings.

The oscillations frequency $f_{osc}$ and/or the oscillation length $T_{osc}= 1/f_{osc}$ and/or the pressure zone of the pre-collapse oscillations state $P_{zone} = [P_{zone1}, P_{zone2}]$ may be estimated experimentally by changing the pump speed S and the pump speed rate R until the pre-collapse oscillations state is reached and the state parameters can be measured.

The present invention relates to a device, preferably a medical apparatus, configured to carry out the method of the present invention, and to a computer readable storage medium storing a computer program configured to carry out said method.

Detailed description and embodiments

Presented method of flow optimization may be achieved in the circuit, especially extracorporeal, comprising drainage (inflow) cannula positioned in the vein, centrifugal pump, oxygenator and the return (outflow) cannula positioned either in the vein or in the artery. Use of a single double-lumen cannula is also conceivable. It is conceivable that any kind of pump providing continuous flow is used.

The circuit design should include a pressure sensor on the inflow line i.e. between the tip of the drainage cannula and the centrifugal pump inlet. Any design of the pressure sensor is applicable. The signal from the pressure sensor is analyzed by the means of an electronic device and a characteristic pattern of pressure curve is identified.

As soon as the system is initiated the pump speed is regulated according to the described algorithm and venous flutter is detected. The detection of the imminent venous collapse indicates maximal possible flow at the current vascular filling status. At the same time, characteristic pattern of the pressure wave can be detected at any flow and at any moment reflecting changing vascular filling status or cannula displacement. Therefore, detection of the characteristic waveform pattern of the drainage pressure can be used as a means of pump control, assessment of the maximal flow limit and as an early alarm of imminent flow/pressure drop.

It is conceivable that the present invention can be used for any device draining blood which can cause vascular collapse i.e. for renal replacement techniques, blood purification and separation, removing intravascular thrombi, etc.

> Fig. 1 shows the pressure changes on the venous line measured during the experiment.
> Fig. 2-4 are characteristic patterns of the pressure changes of fig. 1.

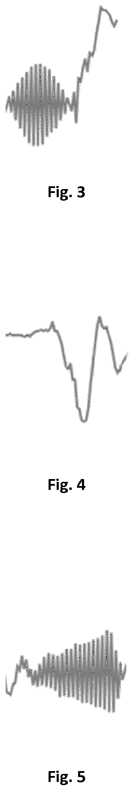

Controlled pump speed increase evokes the characteristic pattern of the pressure waveform. Observed pulsatilities (fig. 3, fig. 4) sustain for a time interval long enough to identify the characteristic frequency of the pump-blood-vessel dynamic system. Further increase of the pump speed causes the flow beyond the inflow and pressure drop followed by collapse manifested in sudden pressure increase observable during the first period of the time series.

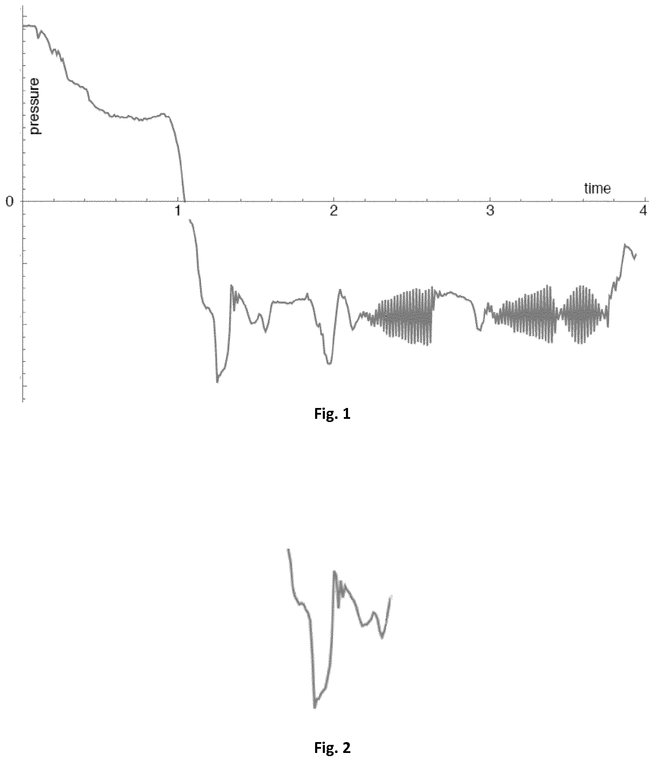

Change of the pump speed leads to the change of the amplitude, exit from the oscillation state and increase of the non-oscillating pressure (Fig. 2). Exceeding pump speed change rate disables the possibility to identify oscillations thus ability to detect pre-collapse state. During the recovery state oscillations are observable. If amplitude is known from prior state pressure zone can be estimated without getting close to critical values.

ANN algorithms allows to detect short oscillations lasting for one or two periods (Fig. 4).

Entering oscillation state causes additional oscillations of the signal easily detected with wavelets algorithms. Pump speed change induces change of amplitude with constant mean value of pressure (Fig. 3, Fig. 5).

Detection of symptomatic pressure waveform is aiming at measuring the frequency, mean value and amplitude of the pressure during steady state of oscillating pump-blood-vessel dynamic system. Thus, the pump speed change must occur within a controlled rate to allow the oscillations to occur in a identifiable form. Furthermore, the mean value stays almost constant during whole oscillations state bringing additional signal for proper detection. Additionally the amplitude of oscillations is related to the pump speed until the collapse occurs. As shown on the Fig. 1 during last observable pulsatility pump speed change induces change of the amplitude followed by exit from the oscillation state and further pressure increase outside oscillation zone. On the contrary, to rapid pump speed increase prohibits the entry into oscillation zone causing collapse eventually followed by residual oscillations shown during first period of observations on Fig. 1.

Given the observable nature of induced phenomena several assumptions on desired features of measurement and detection method can be made:

1. pump speed must be changed slowly enough to allow the measured pressure to remain within the oscillation zone for a time exceeding oscillation cycle length,

2. the ability to detect oscillations depends strongly on the amplitude of the pressure oscillations related to measurement resolution and additional noise originating from patient movements etc. thus increasing the speed of the pump during the oscillations state may help in detecting pulsations,

3. measurement of the pressure mean and amplitude value measured in moving window of the length exceeding expected cycle length allows to detect disruption of relation between pump speed and mean pressure replaced by emerging relation between pump speed and pulsation amplitude during the oscillations state. Such qualitative change of relations between pump speed and features of the pressure signal can be used for precise detection of entering the oscillation state,

4. memorizing oscillation frequency for better efficiency of future detections and estimating oscillation frequency value before the treatment utilizing expected properties of the pump-blood-vessel dynamic system,

5. memorizing oscillation maximum achieved amplitude for limiting the pump speed changes rate,

6. memorizing oscillation mean value and corresponding pump speed for establishing new pump speed limit.

Detection and identification of pre-collapse state the pump-blood-vessel dynamic system procedure steps:

1. estimation of oscillations frequency $f_{osc}$ and oscillation length $T_{osc}=1/f_{osc}$ based on used ECMO devices,

2. estimation of expected maximum pump speed $S_{max}$ and minimal vessel blood pressure $P_{min}$ allowed for given therapy,

3. estimation of maximum rate of non-oscillating pressure change $V_{max}= \Delta P/T_{osc}$ where

a pressure zone of the pre-collapse oscillations state $P_{zone} = [P_{zone1}, P_{zone2}]$ is a pressure range of width $\Delta P = |P_{zone2} - P_{zone1}|$, in which the pre-collapse oscillations state is expected to be observed, and wherein $P_{zone1} > P_{min}$, $P_{zone2} > P_{min}$, and wherein $\Delta P$ is estimated as $\Delta P = a * P_{max}$, with a E (0,1) constant estimated for the equipment used,

4. estimation of maximum rate of pump speed change

$$R_{max}= |k(P_{zone2})-k(P_{zone1})|/T_{osc}$$

where

$k(P)$ - speed-pressure pump characteristics,

5. introducing controlled pump speed increase at rate $R < R_{max}$ with simultaneous control of the pressure $P$ and the pressure change rate $V$ to avoid reaching $P_{min}$ and exceeding $V_{max}$ even if sudden disruptions occur. In case of the pressure change rate exceeding $V_{max}$ the pump speed must be decreased to the point when pressure change rate $V$ did not exceed $V_{max}$ and continue to increase until the $R = R_{max}$ or oscillation state is reached.

6. Oscillation detection based on one or more of following instruments:

a) frequency domain analysis such as FFT for detection of the frequency close to $f_{osc}$ and amplitude changing according to pump speed change

b) time domain analysis of time series for detection of the steady mean value of the pressure with amplitude changing according to pump speed change

c) statistical analysis of residuals filtered from pressure time series with high-pass filter filtering out mean value with time constant $T_{osc}=1/f_{osc}$ utilising the segmentation of the residuals with statistical reasoning on the significance of residuals variation change.

d) wavelets for detecting typical signal shapes occurring during beginning of oscillation state and corresponding to the main frequency of the pump-blood-vessel dynamic system or group frequencies as observable on the Fig. 1,

e) artificial neural networks trained to detect oscillations in the vessel blood pressure time series

7. If oscillations state is reached before maximum pump speed $S_{max}$ is reached, new pump speed limit is set $S_{max}=S_{osc}$, where $S_{osc}$ equals pump speed with oscillations observed with the amplitude lower than maximum with a given ratio.

8. If the pressure $P = P_{min}$ is reached before maximum pump speed $S_{max}$ is reached, new pump speed limit is set $S_{max} = S_{Pmin}$ where $S_{Pmin}$ equals pump speed for which the pressure $P = P_{min}$ is reached.

9. If $S = S_{max}$ is reached then if necessary for the treatment and under strong operator supervision $S_{max}$ can be increased and whole procedure repeated.

Detection and identification procedure can be performed periodically to refresh the maximum pump speed settings. Furthermore if during the treatment any pump speed increase or decrease follows conditions expressed in step 5, then continuous prevention of vessel collapse can be achieved permanently.

Cited prior art

[1] L.A. Baloa et al: "Control of Rotary Heart Assist Devices" Proceedings of 2000 American Control Conference (ACC 2000) Jun. 28-30, 2000 Chicago, IL, USA, BD. 5, 28. June 28, 2000. pp. 2982-2986, XP002487700.

[2] Vollkron et al: "Advanced Suction Detection for an Axial Flow Pump." Artificial Organs, Sep. 2006, p. 665-670, XP 002487701, ISSN: 0160-564X.

[3] US20140200391A1

[4] WO02/35979A2

**Claims**

1. A device or a computer program configured to carry out a method of detecting incipient blood vessel collapse in a circuit comprising means for drainage, preferably a cannula, positioned in said vessel and connected to a pump, wherein the incipient vessel collapse is detected by detecting a pre-collapse oscillation state **characterized by** oscillations of blood pressure of substantially constant frequency and limited amplitude, **characterized in that** in the pre-collapse oscillation state the oscillation frequency $f_{osc}$ is between 0,1Hz and 10Hz and the mean value of the blood pressure is substantially steady.

2. The device or the computer program according to claim 1, wherein in the pre-collapse oscillation state a pump speed change causes change of the amplitude of the oscillations, whereas the mean value of the blood pressure remains substantially steady.

3. The device or the computer program according to claim 1 or 2, wherein a pre-collapse oscillation state is detected by monitoring and analyzing vibrations of the means for drainage, power of the pump and/or a vibro-acoustic characteristic of the pump-blood-vessel dynamic system.

4. The device or the computer program according to claim 1 or 2 or 3, wherein a pre-collapse oscillation state is detected by monitoring the blood pressure on the inlet side of the pump and detecting the oscillations of the blood pressure.

5. The device or the computer program according to claim 3, wherein detection of the oscillations includes one or more of following methods:

   • frequency domain analysis such as Fast Fourier transform (FFT) for detection of the frequency close to $f_{osc}$ and preferably also for detection of the amplitude changes according to pump speed change;
   • time domain analysis of time series for detection of the steady mean value of the blood pressure, and preferably also for detection of the amplitude changes according to pump speed change;
   • statistical analysis of residuals filtered from pressure time series with high-pass filter filtering out mean value with time constant $T_{osc}=1/f_{osc}$ utilising the segmentation of the residuals with statistical reasoning on the significance of residuals variation change;
   • wavelets for detecting typical signal shapes occurring during beginning of the pre-collapse oscillations state and corresponding to the main frequency of the pump-blood-vessel dynamic system or group frequencies;
   • artificial neural networks trained to detect oscillations in the vessel blood pressure time series.

6. The device or the computer program according to any of the preceding claims further configured to carry out a method of optimizing the blood flow of a pump in a circuit comprising means for drainage, preferably a cannula, positioned in said vessel and connected to the pump, **characterized in that** the method comprises the steps of:

   a) estimation of expected maximum pump speed $S_{max}$ and minimum vessel blood pressure

$P_{min}$ allowed for a given therapy,

b) estimation of the oscillation length $T_{osc}= 1/f_{osc}$ and an pressure zone of the pre-collapse oscillations state $P_{zone} = [P_{zone1}, P_{zone2}]$, wherein said pressure zone $P_{zone}$ is a blood pressure range of width $\Delta P = |P_{zone2} - P_{zone1}|$, in which the pre-collapse oscillations state is expected to be observed, and wherein $P_{zone1} > P_{min}$, $P_{zone2} > P_{min}$,

c) estimation of the maximum rate of non-oscillating blood pressure change $V_{max} = \Delta P/T_{osc}$ and the maximum rate of pump speed change rate $R_{max} = |k(P_{zone2}) - k(P_{zone1})|/T_{osc}$, wherein $k(P)$ is a speed-pressure pump characteristics,

d) introducing controlled pump speed increase at rate $R<R_{max}$ with simultaneous control of the blood pressure P and the blood pressure change rate V,

wherein the pump speed S is increased until:

the pump speed $S = S_{max}$ is reached, or
the blood pressure $P = P_{min}$ is reached, or
an incipient blood vessel collapse is detected, or
the blood pressure change rate V exceeds $V_{max}$,

and wherein the pump speed is temporarily decreased if the pressure change rate V exceeds $V_{max}$ until the pressure change rate V does not exceed $V_{max}$, and then is further increased,

e) setting new maximum pump speed $S_{max} = S_{osc}$ if the incipient blood vessel collapse is detected for the pump speed $S = S_{osc}$ before maximum pump speed $S_{max}$ is reached, or setting new maximum pump speed $S_{max} = S_{Pmin}$ if the blood pressure $P = P_{min}$ is reached for the pump speed $S = S_{Pmin}$ before maximum pump speed $S_{max}$ is reached.

7. The device or the computer program according to claim 6, wherein the method step e) further comprises the step of increasing maximum pump speed $S_{max}$ with a predetermined value, if $S = S_{max}$ is reached during the method step d) .

8. The device or the computer program according to claim 6 or 7, wherein the steps d) and e) are performed periodically or on demand of the operator to refresh the maximum pump speed settings.

9. The device or the computer program according to claim 6 or 7 or 8, wherein the oscillations frequency $f_{osc}$ and/or the oscillation length $T_{osc}= 1/f_{osc}$ and/or the pressure zone of the pre-collapse oscillations state $P_{zone} = [P_{zone1}, P_{zone2}]$ is estimated experimentally by changing the pump speed S and the pump speed rate R until the pre-collapse oscillations state is reached and the state parameters can be measured.

10. The device according to any of the preceding claims, wherein the device is a medical apparatus.

11. A computer readable storage medium storing the computer program according to any of the claims from 1 to 9.

12. A method of detecting incipient blood vessel collapse in a circuit comprising means for drainage, preferably a cannula, positioned in said vessel and connected to a pump, wherein the incipient vessel collapse is detected by detecting a pre-collapse oscillation state **characterized by** oscillations of blood pressure of substantially constant frequency and limited amplitude, **characterized in that** in the pre-collapse oscillation state the oscillation frequency $f_{osc}$ is between 0,1Hz and 10Hz and the mean value of the blood pressure is substantially steady.

**Patentansprüche**

1. Vorrichtung oder Computerprogramm, die bzw. das so konfiguriert ist, dass sie bzw. es ein Verfahren zum Erkennen eines beginnenden Blutgefäßkollapses in einem Kreislauf durchführt, der eine Einrichtung zur Drainage, vorzugsweise eine Kanüle, umfasst, die in dem Gefäß angeordnet und mit einer Pumpe verbunden ist, wobei der beginnende Gefäßkollaps durch Erkennen eines Oszillationszustands vor dem Kollaps erkannt wird, der durch Oszillationen des Blutdrucks mit im Wesentlichen konstanter Frequenz und begrenzter Amplitude **gekennzeichnet ist, dadurch** gekennzeichnet, dass in dem Oszillationszustands vor dem Kollaps die Oszillationsfrequenz $f_{osc}$ zwischen 0,1 Hz und 10 Hz liegt und der Mittelwert des Blutdrucks im Wesentlichen konstant ist.

2. Die Vorrichtung oder das Computerprogramm nach Anspruch 1, wobei im Zustand vor dem Kollaps der Oszillation eine Änderung der Pumpendrehzahl eine Änderung der Amplitude der Oszillationen bewirkt, während der Mittelwert des Blutdrucks im Wesentlichen konstant bleibt.

3. Die Vorrichtung oder das Computerprogramm nach Anspruch 1 oder 2, wobei ein Oszillationszustand vor dem Kollaps durch Überwachen und Analysieren von Oszillationen des Drainagemittels, der Leistung der Pumpe und/oder einer vibroakustischen Charakteristik des dynamischen Systems Pumpe-Blutgefäß erkannt wird.

4. Die Vorrichtung oder das Computerprogramm nach Anspruch 1 oder 2 oder 3, wobei ein Oszillationszustand vor dem Kollaps durch Überwachung des Blutdrucks auf der Einlassseite der Pumpe und Erkennung der Oszillationen des Blutdrucks erkannt wird.

**5.** Die Vorrichtung oder das Computerprogramm nach Anspruch 3, wobei die Erfassung der Oszillationen eines oder mehrere der folgenden Verfahren umfasst:

- Analyse im Frequenzbereich, wie z. B. die schnelle Fourier-Transformation (FFT), zur Erkennung der Frequenz nahe $f_{osc}$ und vorzugsweise auch zur Erkennung der Amplitudenänderungen entsprechend der Änderung der Pumpendrehzahl;

- Analyse der Zeitreihen im Zeitbereich zur Ermittlung des konstanten Mittelwerts des Blutdrucks und vorzugsweise auch zur Ermittlung der Amplitudenänderungen entsprechend der Änderung der Pumpendrehzahl;

- statistische Analyse der Residuen, die aus der Druckzeitreihe mit Hochpass gefiltert wurden, wobei der Mittelwert mit der Zeitkonstante $T_{osc}=1/f_{osc}$ herausgefiltert wurde, unter Verwendung der Segmentierung der Residuen mit statistischen Schlussfolgerungen über die Bedeutung der Veränderung der Residuen;

- Wavelets zur Erkennung typischer Signalformen, die zu Beginn des Oszillationszustand vor dem Kollaps auftreten und der Hauptfrequenz des dynamischen Systems Pumpe-Blutgefäß oder Gruppenfrequenzen entsprechen;

- künstliche neuronale Netze, die darauf trainiert sind, Oszillationen in den Zeitreihen des Gefäßblutdrucks zu erkennen.

**6.** Die Vorrichtung oder das Computerprogramm nach einem der vorhergehenden Ansprüche, die bzw. das außerdem so konfiguriert ist, dass sie bzw. es ein Verfahren zur Optimierung des Blutflusses einer Pumpe in einem Kreislauf durchführt, der ein Mittel zur Drainage, vorzugsweise eine Kanüle, umfasst, das in dem Gefäß angeordnet und mit der Pumpe verbunden ist, **dadurch gekennzeichnet, dass** das Verfahren die folgenden Schritte umfasst:

a) Schätzung der zu erwartenden maximalen Pumpendrehzahl $S_{max}$ und des minimal zulässigen Gefäßblutdrucks $P_{min}$ für eine bestimmte Therapie,

b) Schätzung der Oszillationslänge $T_{osc}= 1/f_{osc}$ und einer Druckzone des Oszillationszustands vor dem Kollaps $P_{zone} = [P_{zone1}, P_{zone2}]$, wobei die Druckzone $P_{zone}$ ein Blutdruckbereich der Breite $\Delta P = |P_{zone2} - P_{zone1}|$ ist, in dem der Oszillationszustand vor dem Kollaps voraussichtlich beobachtet wird, und WObei $P_{zone1} > P_{min}$,

$P_{zone2} > P_{min}$,

c) Schätzung der maximalen Rate der nicht oszillierenden Blutdruckänderung $V_{max} = \Delta P/T_{osc}$ und der maximalen Rate der Pumpendrehzahländerungsrate $R_{max} = |k(P_{zone2}) - k(P_{zone1})|/ T_{ose}$, wobei $k(P)$ eine Drehzahl-Druck-Pumpenkennlinie ist,

d) Einführung einer kontrollierten Erhöhung der Pumpendrehzahl mit einer Rate $R < R_{max}$ bei gleichzeitiger Kontrolle des Blutdrucks $P$ und der Blutdruckänderungsrate $V$, wobei die Pumpendrehzahl $S$ erhöht wird, bis:

die Pumpendrehzahl $S = S_{max}$ erreicht ist, oder

der Blutdruck $P = P_{min}$ erreicht ist, oder ein beginnender Blutgefäßkollaps festgestellt wird, oder

die Blutdruckänderungsrate $V$ $V_{max}$ überschreitet

und wobei die Pumpendrehzahl vorübergehend verringert wird, wenn die Druckänderungsrate $V$ $V_{max}$ übersteigt, bis die Druckänderungsrate $V$ $V_{max}$ nicht mehr übersteigt, und dann weiter erhöht wird,

e) Einstellung einer neuen maximalen Pumpendrehzahl $S_{max} = S_{osc}$, wenn bei der Pumpendrehzahl $S = S_{osc}$ der beginnende Blutgefäßkollaps festgestellt wird, bevor die maximale Pumpendrehzahl $S_{max}$ erreicht ist, oder Einstellung einer neuen maximalen Pumpendrehzahl $S_{max} = S_{Pmin}$, wenn bei der Pumpendrehzahl $S = S_{Pmin}$ der Blutdruck $P =_{Pmin}$ erreicht wird, bevor die maximale Pumpendrehzahl $S_{max}$ erreicht ist.

**7.** Die Vorrichtung oder das Computerprogramm nach Anspruch 6, wobei der Verfahrensschritt e) ferner den Schritt der Erhöhung der maximalen Pumpendrehzahl $S_{max}$ um einen vorgegebenen Wert umfasst, wenn $S = S_{max}$ während des Verfahrensschritts d) erreicht wird.

**8.** Die Vorrichtung oder das Computerprogramm nach Anspruch 6 oder 7, wobei die Schritte d) und e) periodisch oder auf Anforderung des Bedieners durchgeführt werden, um die Einstellungen der maximalen Pumpendrehzahl zu aktualisieren.

**9.** Die Vorrichtung oder das Computerprogramm nach Anspruch 6 oder 7 oder 8, wobei die Oszillationsfrequenz $f_{osc}$ und/oder die Oszillationslänge $T_{ose}= 1/f_{osc}$ und/oder die Druckzone des Oszillationszustand vor dem Kollaps $P_{zone}= [P_{zone1}, P_{zone2}]$ experimentell abgeschätzt wird, indem die Pumpendrehzahl $S$ und die Pumpendrehzahlrate $R$ so lange verändert werden, bis der Oszillationszustand vor dem Kollaps erreicht ist und die Zustandsparameter gemessen werden können.

10. Die Vorrichtung nach einem der vorhergehenden Ansprüche, wobei die Vorrichtung ein medizinisches Gerät ist.

11. Computerlesbares Speichermedium, das das Computerprogramm nach einem der Ansprüche 1 bis 9 speichert.

12. Verfahren zum Nachweisen eines beginnenden Blutgefäßkollapses in einem Kreislauf, der eine Einrichtung zur Drainage, vorzugsweise eine Kanüle, aufweist, die in dem Gefäß angeordnet und mit einer Pumpe verbunden ist, wobei der beginnende Gefäßkollaps durch Nachweisen eines Oszillationszustandes vor dem Kollaps nachgewiesen wird, der durch Oszillationen des Blutdrucks mit im wesentlichen konstanter Frequenz und begrenzter Amplitude **gekennzeichnet ist, dadurch** gekennzeichnet, dass in dem Oszillationszustand vor dem Kollaps die Oszillationsfrequenz $f_{osc}$ zwischen 0,1 Hz und 10 Hz liegt und der Mittelwert des Blutdrucks im wesentlichen konstant ist.

**Revendications**

1. Un dispositif ou un programme informatique configuré pour mettre en oeuvre une méthode de détection d'un effondrement naissant des vaisseaux sanguins dans un circuit comprenant des moyens de drainage, de préférence une canule, positionnés dans ledit vaisseau et reliés à une pompe, dans lequel l'effondrement naissant des vaisseaux est détecté en détectant un état d'oscillation préalable à l'effondrement **caractérisé par** des oscillations de la pression sanguine de fréquence sensiblement constante et d'amplitude limitée, **caractérisé en ce que** dans l'état d'oscillation préalable à l'effondrement, la fréquence d'oscillation $f_{osc}$ est comprise entre 0,1 Hz et 10 Hz et la valeur moyenne de la pression sanguine est sensiblement stable.

2. Le dispositif ou le programme informatique selon la revendication 1, dans lequel dans l'état d'oscillation préalable à l'effondrement, le changement de vitesse de la pompe provoque un changement de l'amplitude des oscillations, tandis que la valeur moyenne de la pression sanguine reste sensiblement stable.

3. Le dispositif ou le programme informatique selon la revendication 1 ou 2, dans lequel un état d'oscillation préalable à l'effondrement est détecté en surveillant et en analysant les vibrations des moyens de drainage, la puissance de la pompe et/ou une caractéristique vibro-acoustique du système dynamique pompe-sang-vaisseau.

4. Le dispositif ou le programme informatique selon la revendication 1 ou 2, ou 3, dans lequel un état d'oscillation préalable à l'effondrement est détecté en surveillant la pression sanguine du côté entrée de la pompe et en détectant les oscillations de la pression sanguine.

5. Le dispositif ou le programme informatique selon la revendication 3, dans lequel la détection des oscillations comprend une ou plusieurs des méthodes suivantes :

• analyse dans le domaine des fréquences, telle que la transformée de Fourier rapide (FFT), pour détecter la fréquence proche de $f_{osc}$ et, de préférence, également pour détecter les changements d'amplitude en fonction du changement de vitesse de la pompe ;
• analyse dans le domaine temporel des séries temporelles pour détecter la valeur moyenne stable de la pression sanguine, et de préférence aussi pour détecter des changements d'amplitude en fonction du changement de vitesse de la pompe ;
• analyse statistique des résidus filtrés à partir des séries temporelles de pression avec un filtre passe-haut filtrant la valeur moyenne avec une constante de temps $T_{osc}=1/f_{osc}$ en utilisant la segmentation des résidus avec un raisonnement statistique sur l'importance du changement de variation des résidus ;
• ondelettes pour détecter des formes de signaux typiques apparaissant au début de l'état d'oscillations préalable à l'effondrement et correspondant à la fréquence principale du système dynamique pompe-sang-vaisseau ou à des fréquences de groupe ;
• réseaux neuronaux artificiels entraînés à détecter des oscillations dans les séries temporelles de la pression sanguine des vaisseaux.

6. Le dispositif ou le programme informatique selon l'une quelconque des revendications précédentes configuré en outre pour mettre en oeuvre une méthode d'optimisation du débit sanguin d'une pompe dans un circuit comprenant des moyens de drainage, de préférence une canule, positionnés dans ledit vaisseau et reliés à la pompe, **caractérisé en ce que** la méthode comprend les étapes suivantes :

a) estimation de la vitesse maximale $S_{max}$ de la pompe et de la pression sanguine minimale $P_{min}$ du vaisseau autorisées pour un traitement donné,
b) estimation de la longueur d'oscillation $T_{osc}=1/f_{osc}$ et d'une zone de pression de l'état d'oscillations préalable à l'effondrement $P_{zone}$ = $[P_{zone1}, P_{zone2}]$, dans laquelle ladite zone de pression Pzone est une plage de pression sanguine de largeur $\Delta P = |P_{zone2} - P_{zone1}|$, dans laquelle l'état d'oscillations préalable à l'effondrement est censé être observé, et dans laquelle $P_{zone1} > P_{min}$, $P_{zone2} > P_{min}$,

c) estimation du taux maximal de changement de pression sanguine non oscillante $V_{max}$ = $\Delta P/T_{osc}$ et du taux maximal de changement de vitesse de la pompe $R_{max}$ = $|k(P_{zone2})$ - $k(P_{zone1})|/ T_{osc}$, où $k(P)$ est une caractéristique vitesse-pression de la pompe,

d) introduction d'une augmentation contrôlée de la vitesse de la pompe au taux $R<R_{max}$ avec contrôle simultané de la pression sanguine P et du taux de changement V de la pression sanguine,

dans laquelle la vitesse S de la pompe est augmentée jusqu'à ce que :

la vitesse de la pompe $S = S_{max}$ soit atteinte ou

la pression artérielle $P = P_{min}$ soit atteinte, ou un effondrement naissant des vaisseaux sanguins soit détecté, ou

le taux de variation V de la pression sanguine dépasse $V_{max}$,

et dans laquelle la vitesse de la pompe est temporairement réduite si le taux de changement de pression V dépasse $V_{max}$ jusqu'à ce que le taux de changement de pression V ne dépasse pas $V_{max}$, et est ensuite encore augmenté,

e) réglage d'une nouvelle vitesse maximale de la pompe $S_{max} = S_{osc}$ si l'effondrement naissant des vaisseaux sanguins est détecté pour la vitesse de la pompe $S = S_{osc}$ avant que la vitesse maximale de la pompe $S_{max}$ soit atteinte, ou réglage d'une nouvelle vitesse maximale de la pompe $S_{max} = S_{Pmin}$ si la pression sanguine P $= P_{min}$ est atteinte pour la vitesse de la pompe S $= S_{Pmin}$ avant que la vitesse maximale de la pompe $S_{max}$ soit atteinte.

7. Le dispositif ou le programme informatique selon la revendication 6, dans lequel l'étape e) de la méthode comprend en outre l'étape d'augmentation de la vitesse maximale de la pompe $S_{max}$ avec une valeur prédéterminée, si $S = S_{max}$ est atteint pendant l'étape d) de la méthode.

8. Le dispositif ou le programme informatique selon la revendication 6 ou 7, dans lequel les étapes d) et e) sont exécutées périodiquement ou à la demande de l'opérateur pour rafraîchir les réglages de vitesse maximale de la pompe.

9. Le dispositif ou le programme informatique selon la revendication 6 ou 7, ou 8, dans lequel la fréquence d'oscillations $f_{osc}$ et/ou la longueur d'oscillation $T_{osc}$= $1/f_{osc}$, et/ou la zone de pression de l'état d'oscillations préalable à l'effondrement $P_{zone}$ = $[P_{zone1}, P_{zone2}]$ est estimée expérimentalement en changeant la vitesse de la pompe S et le taux de vitesse de la pompe R jusqu'à ce que l'état d'oscillations préalable à l'effondrement soit atteint et que les paramètres d'état puissent être mesurés.

10. Le dispositif selon l'une quelconque des revendications précédentes, dans lequel le dispositif est un appareil médical.

11. Un support de stockage lisible par ordinateur stockant le programme informatique selon l'une quelconque des revendications de 1 à 9.

12. Une méthode de détection d'un effondrement naissant des vaisseaux sanguins dans un circuit comprenant des moyens de drainage, de préférence une canule, positionnés dans ledit vaisseau et reliés à une pompe, dans laquelle l'effondrement naissant des vaisseaux est détecté en détectant un état d'oscillation préalable à l'effondrement **caractérisé par** des oscillations de la pression sanguine de fréquence sensiblement constante et d'amplitude limitée, **caractérisée en ce que** dans l'état d'oscillation préalable à l'effondrement, la fréquence d'oscillation $f_{osc}$ est comprise entre 0,1 Hz et 10 Hz et la valeur moyenne de la pression sanguine est sensiblement stable.

**Fig. 1**

**Fig. 2**

**Fig. 3**

**Fig. 4**

**Fig. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20140200391 A1

- WO 0235979 A2

### Non-patent literature cited in the description

- **L.A. BALOA et al.** Control of Rotary Heart Assist Devices. *Proceedings of 2000 American Control Conference (ACC 2000),* 28 June 2000, vol. 5 (28), 2982-2986

- **VOLLKRON et al.** Advanced Suction Detection for an Axial Flow Pump. *Artificial Organs,* September 2006, ISSN 0160-564X, 665-670